# EUROPÄISCHE PATENTSCHRIFT

(11) **EP 3 513 771 B1**
(45) Veröffentlichungstag und Bekanntmachung des Hinweises auf die Patenterteilung: **26.11.2025**
(21) Anmeldenummer: 18153076.7
(22) Anmeldetag: 23.01.2018
(51) Int. Cl.: A61F 5/41, A61H 19/00

(54) **SCHWELLKÖRPER-TRAINIERVORRICHTUNG**
ERECTILE TISSUE TRAINING DEVICE
DISPOSITIF D'ENTRAÎNEMENT POUR CORPS CAVERNEUX

(43) Veröffentlichungstag der Anmeldung: 24.07.2019
(73) Patentinhaber: Pignitter, Bernd, 8046 Graz (AT)
(72) Erfinder: Pignitter, Bernd, 8046 Graz (AT)
(74) Vertreter: Rothkopf, Ferdinand

(56) Entgegenhaltungen:
- EP-A1- 2 191 805
- EP-A1- 2 583 658
- CN-Y- 2 880 021
- US-A1- 2015 196 454

## Beschreibung

### Hintergrund der Erfindung

Die Erfindung betrifft eine Schwellkörper-Trainiervorrichtung mit mindestens einem Druck-Element und einem Gegendruck-Element.

Vorrichtungen zum Trainieren menschlicher Körperteile, um diese zu vergrößern, sind allgemein bekannt. Bei einem Penis wird dabei speziell ein Schwellkörper trainiert, um diesen vor allem hinsichtlich seiner Länge zu vergrößern. Der Schwellkörper ist grundsätzlich ein sich mit Blut füllendes und dadurch physische Aufgaben erfüllendes Gefäßgeflecht. Dabei ist der Schwellkörper von einer dicken Bindegewebskapsel umgeben ist, die dafür sorgt, dass sich der Penis bei einer Erektion versteift und verlängert. Aus dem Stand der Technik sind Vorrichtungen bekannt, bei denen ein stetiger Zug, Druck oder Unterdruck bzw. Vakuum an den Schwellkörper angelegt wird, um dadurch eine dauerhafte oder zumindest vorübergehende Vergrößerung des Penis zu erreichen. Solche veröffentlichten Vorrichtungen sind beispielsweise DE 102007020236 B3, DE 4341790 A1 und EP 1779822 B1. Bei solchen Vorrichtungen erfolgt eine ununterbrochene Belastung mindestens eines Teils des Schwellkörpers während der Anwendung, wodurch negative gesundheitliche Folgen, zum Beispiel aufgrund unzureichender Blutversorgung des Schwellkörpers, nicht ausgeschlossen werden können.

Aus EP 2 191 805 A1, die als nächstliegender Stand der Technik angesehen werden kann, ist ein Massagegerät mit einer länglichen Massageausnehmung zur Aufnahme eines männlichen Geschlechtsteils bekannt, wobei zur Massage eines in der länglichen Massageausnehmung aufgenommenen männlichen Geschlechtsteils eine sich entlang der Längsachse der Massageausnehmung erstreckende Antriebseinrichtung vorgesehen ist. Die Antriebseinrichtung ist unter anderem für ein wellenartiges Verformen der länglichen Massageausnehmung ausgebildet, insbesondere einer entlang der Längsachse der länglichen Massageausnehmung fortschreitenden Welle

### Zugrundeliegende Aufgabe

Der Erfindung liegt die Aufgabe zugrunde, eine Vorrichtung zur Vergrößerung eines Schwellkörpers zu schaffen, die die Gefahr von negativen gesundheitlichen Folgen verringert oder zumindest einen Nachteil der Vorrichtungen aus dem Stand der Technik überwindet.

### Erfindungsgemäße Lösung

Diese Aufgabe ist erfindungsgemäß mit einer Schwellkörper-Trainiervorrichtung gemäß Anspruch 1 gelöst. Dabei ist das mindestens eine Druck-Element dafür vorgesehen, in Richtung des Gegendruck-Elements an einen zwischen dem mindestens einen Druck-Element und dem Gegendruck-Element positionierten Schwellkörper einen wiederkehrenden, insbesondere periodisch wiederkehrenden Druck, anzulegen. Mit dem periodisch wiederkehrenden Druck ist hier gemeint, dass sich der Druck regelmäßig wiederholt. Der Schwellkörper wird also nicht dauerhaft zusammengedrückt, sondern in wiederkehrenden Abständen. Dadurch wird der Schwellkörper trainiert und merkbar vergrößert. Gleichzeitig wird eine Blutversorgung bzw. ein Blutzufluss und -abfluss gewährleistet und noch zusätzlich angeregt. Somit wird das Risiko von gesundheitlichen Schäden gegenüber einer stetigen Druckbelastung deutlich herabgesetzt. Ein weiterer vorteilhafter Effekt ist eine Verstärkung der Erektion, welche sich bei Anwendung der Schwellkörper-Trainiervorrichtung einstellt.

Gemäß der Erfindung ist das mindestens eine Druck-Element dazu vorgesehen, den wiederkehrenden Druck auf den Schwellkörper in Form einer Wellenbewegung anzulegen. Mit der Wellenbewegung ist hier eine sich räumlich ausbreitende Veränderung einer physikalischen Größe, hier insbesondere eines Drucks, gemeint. Dabei erfolgt die Wellenbewegung erfindungsgemäß in Richtung einer Eichel, die ein vorderes Penisende bildet. Dabei wird der Schwellkörper gedehnt und somit vergrößert.

Bei einer vorteilhaften Weiterbildung der Erfindung ist die Schwellkörper-Trainiervorrichtung von einem Gehäuse mit einer ersten Seitenwand und einer zweiten Seitenwand umgeben, wobei die erste Seitenwand eine erste Wandöffnung und die zweite Seitenwand eine zweite Wandöffnung aufweist. Die Wandöffnungen sind dafür vorgesehen, den Penis durch beide Seitenwände zu schieben und dadurch passgenau in der Schwellkörper-Trainiervorrichtung zu positionieren. Dabei sind die Wandöffnungen vorzugsweise so angeordnet, dass sich das Gegendruck-Element an einer Oberseite der Wandöffnungen befindet. Das Gehäuse ermöglicht ein sicheres Positionieren verschiedener Komponenten der Schwellkörper-Trainiervorrichtung, in dem diese in dem Gehäuse sicher befestigt werden. Zudem bietet das Gehäuse Schutz vor Verletzungen von menschlichen Körperteilen an den Komponenten der Schwellkörper-Trainiervorrichtung, die nicht für einen Kontakt mit solchen Körperteilen vorgesehen sind.

In einer weiteren vorteilhaften Weiterbildung der Erfindung ist das Gegendruck-Element in seinem Abstand zu dem mindestens einen Druck-Element verstellbar, um den Schwellkörper zu fixieren. Der Schwellkörper wird also zwischen zwei Druck-Elementen leicht eingespannt. Somit wird der Druck wirksam an den Schwellkörper angelegt und ein Verrutschen des Schwellkörpers innerhalb der Schwellkörper-Trainiervorrichtung verhindert. Zudem kann auf diese Weise die Intensität des anzulegenden Drucks an den Schwellkörper eingestellt werden.

Bevorzugt hat das mindestens eine Druck-Element eine Druck-Element-Oberseite und das Gegendruck-Element eine der Druck-Element-Oberseite gegenüberliegende Gegendruck-Element-Unterseite, wobei die Druck-Element-Oberseite und/oder die Gegendruck-Element-Unterseite jeweils eine Innenwölbung aufweisen. Die sich gegenüberliegenden Seiten des mindestens einen Druck-Elements und/oder des Gegendruckelements sind also konkav geformt. Dadurch wird ein zentrales Anliegen des Schwellkörpers unterstützt und ein seitlichen Abrutschen desselben verhindert.

Das mindestens eine Druck-Element ist bevorzugt mit einer Führ-Einrichtung verbunden, die ein Bewegen des mindestens einen Druck-Elements in Richtung des Gegendruck-Elements und zurück erlaubt. Die Führ-Einrichtung ist dabei vorzugsweise am Gehäuse unterhalb der Wandöffnungen befestigt und sorgt dafür, dass sich das mindestens eine Druck-Element geradlinig auf das Gegendruck-Element zu- und von diesem wegbewegt, also vertikal verschiebbar ist. Mittels der Führ-Einrichtung wird eine stabile Bewegung des mindestens einen Druck-Elements, insbesondere in zwei Richtungen, und somit ein wiederkehrender Druck auf den Schwellkörper gewährleistet.

Ferner weist das mindestens eine Druck-Element vorteilhaft eine Druck-Element-Unterseite auf, wobei die Druck-Element-Unterseite vorzugsweise U-förmig gestaltet ist. Die Druck-Element-Unterseite ermöglicht das Anlegen eines Drucks zum Bewegen des Druck-Elements in Richtung des Gegendruck-Elements. Die U-Form dient dabei als Führungsschiene für einen druckausübenden Gegenstand, verhindert dabei, dass dieser Gegenstand seitlich abrutscht und ermöglicht ein Gleiten des Gegenstands auf der Druck-Element-Unterseite. Somit ist das mindestens eine Druck-Element flexibel mit verschiedensten Gegenständen zum Druckausüben kombinierbar.

Vorteilhaft weist die Schwellkörper-Trainiervorrichtung ferner mindestens einen Kipphebel auf, der zum Anlegen eines Drucks an das mindestens eine Druck-Element vorgesehen ist. Ein Kipphebel ist als zweiseitiger Hebel ausgeführt und dient grundsätzlich dazu, die Richtung einer Kraft zu ändern. Somit ist das Anlegen des Drucks mittels einer Kraftausübung in oder von verschiedenen Richtungen möglich, wobei zusätzlich eine Hebelwirkung ausgenutzt werden kann. Außerdem erlaubt eine solche Kraftumlenkung mehr Flexibilität bei der Bauform der Schwellkörper-Trainiervorrichtung.

Dabei ist der mindestens eine Kipphebel bevorzugt L-förmig mit einem Horizontal-Abschnitt und einem Vertikal-Abschnitt gestaltet, wobei der Horizontal-Abschnitt vorzugsweise zum Drücken des mindestens einen Druck-Elements in Richtung des Gegendruck-Elements vorgesehen ist. Bei dem L-förmigen Kipphebel wird mittels einer horizontal wirkenden Kraft auf den Vertikal-Abschnitt eine vertikale Kraft mittels des Horizontal-Abschnitts auf das mindestens eine Druck-Element übertragen. Der L-förmige Kipp-Hebel erzielt die gleichen Vorteile wie der oben beschriebene Kipphebel, lässt sich dabei aber besonders einfach herstellen.

Darauf aufbauend ist der mindestens eine Kipphebel in vorteilhafter Weise mit einer Drehachse verbunden, wobei die Drehachse rechtwinklig zum Horizontal-Abschnitt und zum Vertikal-Abschnitt des mindestens einen Kipphebels angeordnet ist. Anders ausgedrückt, wenn in einem dreidimensionalen Koordinatensystem der Horizontal-Abschnitt die x-Achse und der Vertikal-Abschnitt die y-Achse bilden, dann bildet die Drehachse die z-Achse. Dabei ist die Drehachse am Gehäuse sicher befestigt. Mithilfe der verbundenen Drehachse ist ein Drehpunkt des Kipphebels festgelegt und der Kipphebel fest in der Schwellkörper-Trainiervorrichtung verankert.

Ferner weist die Schwellkörper-Trainiervorrichtung bevorzugt eine Zieh-Einrichtung auf, wobei die Zieh-Einrichtung mittels eines Verbindungs-Elements mit dem mindestens einen Kipphebel verbunden ist. Der mindestens eine Kipphebel ist also dadurch bewegbar, dass die Zieh-Einrichtung mit dem Verbindungs-Element eine Zugkraft auf den Kipphebel ausübt, um diesen zu verdrehen. Dabei ist das Verbindungs-Element vorzugsweise mit dem Vertikal-Abschnitt des Kipphebels verbunden. Somit wird eine Kraft effizient von der Zieh-Einrichtung bis letztendlich zu dem mindestens einen Druck-Element übertragen und dabei verstärkt.

Dabei ist die Zieh-Einrichtung vorteilhaft als eine Rotationswelle mit mindestens einem Verbindungsbolzen gestaltet, wobei der Verbindungsbolzen mit dem Verbindungs-Element verbunden ist und eine Bolzenachse des mindestens einen Verbindungsbolzen außerhalb einer Rotationsachse der Rotationswelle angeordnet ist. Beim Drehen der Rotationswelle bewegt sich der mindestens eine Verbindungsbolzen somit auf einer Kreislinie. Das mit dem Verbindungsbolzen verbundene Verbindungs-Element, welches vorzugsweise als ein Seilzug gestaltet ist, überträgt eine Kreisbewegung des Verbindungsbolzens in eine Kippel- bzw. Wackelbewegung des Kipphebels. Der Seilzug ist dabei frei beweglich über den Verbindungsbolzen geführt. Auf diese Weise wird letztendlich ein Wiederkehren des an den Schwellkörper anzulegenden Drucks mittels des mindestens einen Druck-Elements realisiert.

Besonders bevorzugt weist die Rotationswelle mehrere Verbindungsbolzen auf, die um die Rotationsachse der Rotationswelle derart angeordnet sind, dass sie eine Spiralform bilden. Dabei ist die Anzahl der Druck-Elemente, Kipphebel und Verbindungs-Elemente vorzugsweise gleich der Anzahl der Verbindungsbolzen. Dadurch wird erreicht, dass bei einer vollständigen 360°-Drehung der Rotationswelle der Abstand eines jeden Druck-Elements zum Gegendruck-Element einmal minimiert und einmal maximiert wird. Dadurch erfährt der Schwellkörper einen wiederkehrenden Druck im Sinne einer Wellenbewegung, die sich vorzugsweise in Richtung der Eichel bewegt. Der Schwellkörper wird dabei gedehnt und der Penis merkbar vergrößert. Zudem stellt sich eine Verstärkung der Erektion ein.

Ferner sind weitere Ausführungsformen möglich, welche die Schwellkörper-Trainiervorrichtung noch effizienter machen.

So weist die Schwellkörper-Trainiervorrichtung vorteilhaft mindestens ein erstes Druck-Element und ein zweites Druck-Element auf, die dazu vorgesehen sind, nacheinander jeweils einen Druck an den Schwellkörper anzulegen, wobei zuerst das erste Druck-Element einen ersten Druck an einer ersten Stelle des Schwellkörpers und danach das zweite Druck-Element einen zweiten Druck an einer zweiten Stelle des Schwellkörpers anlegt. Hinsichtlich der Vergrößerung des Schwellkörpers und der Verstärkung der Erektion werden dabei die gleichen Vorteile wie oben beschrieben erzielt.

In einer vorteilhaften Weiterbildung sind die Wandöffnungen mit einer Manschette, besonders bevorzugt mit einer Latex-Manschette, versehen. Die Manschetten, besonders die Latex-Manschetten, machen die Nutzung der Schwellkörper-Trainiervorrichtung für einen Nutzer deutlich angenehmer und schützen den Penis vor einer Berührung mit harten Kanten der Wandöffnungen.

In einer weiteren vorteilhaften Weiterbildung der Erfindung ist der Horizontal-Abschnitt des Kipphebels so positioniert, dass zumindest ein Ende dieses Horizontal-Abschnitts in der U-förmigen Druck-Element-Unterseite eingebettet ist. Ein sicheres Verschieben eines Kontaktpunkts oder einer Kontaktfläche des Kipphebels auf der Druck-Element-Unterseite wird somit gewährleistet.

Ferner weist die Führ-Einrichtung vorzugsweise mindestens eine Gleithülse auf. Die mindestens eine Gleithülse ist eine fest montierte Komponente der Führ-Einrichtung, durch welche eine Komponente des mindestens einen Druck-Elements geführt wird, sodass diese Komponente beim Bewegen durch die Gleithülse hindurch gleitet. Die geradlinige Bewegung des mindestens einen Druck-Elements wird dadurch zusätzlich abgesichert.

### Kurzbeschreibung der Zeichnungen

Nachfolgend wird ein Ausführungsbeispiel der erfindungsgemäßen Lösung anhand der beigefügten schematischen Zeichnungen näher erläutert. Es zeigt:
- Fig. 1: einen Längsschnitt einer Schwellkörper-Trainiervorrichtung gemäß der Erfindung,
- Fig. 2: den Schnitt II - II gemäß Fig. 1,
- Fig. 3: das Detail III gemäß Fig. 1,
- Fig. 4: das Detail IV gemäß Fig. 1,
- Fig. 5: das Detail V gemäß Fig. 1,
- Fig. 6: das Detail VI gemäß Fig. 1 und
- Fig. 7: den Schnitt VII - VII gemäß Fig. 6.

### Detaillierte Beschreibung des Ausführungsbeispiels

Bevor die Vorrichtungen im Detail beschrieben wird, sei explizit darauf hingewiesen, dass diese Erfindung nicht auf die beschriebenen Vorrichtungen beschränkt ist. Es sei auch darauf hingewiesen, dass die in der Beschreibung verwendete Terminologie nur dazu dient, die bestimmten Ausführungsformen zu beschreiben, und nicht dazu gedacht ist, den Umfang der vorliegenden Erfindung zu beschränken.

In den folgenden Figurenbeschreibungen werden einige Komponenten bzw. Begriffe mit Bezugszeichen auch dann verwendet, wenn sie nicht in der jeweils zu beschreibenden Figur enthalten sind, aber nur dann, wenn diese Bezugszeichen in einer vorhergehenden Figur und Figurenbeschreibung mindestens einmal verwendet wurden.

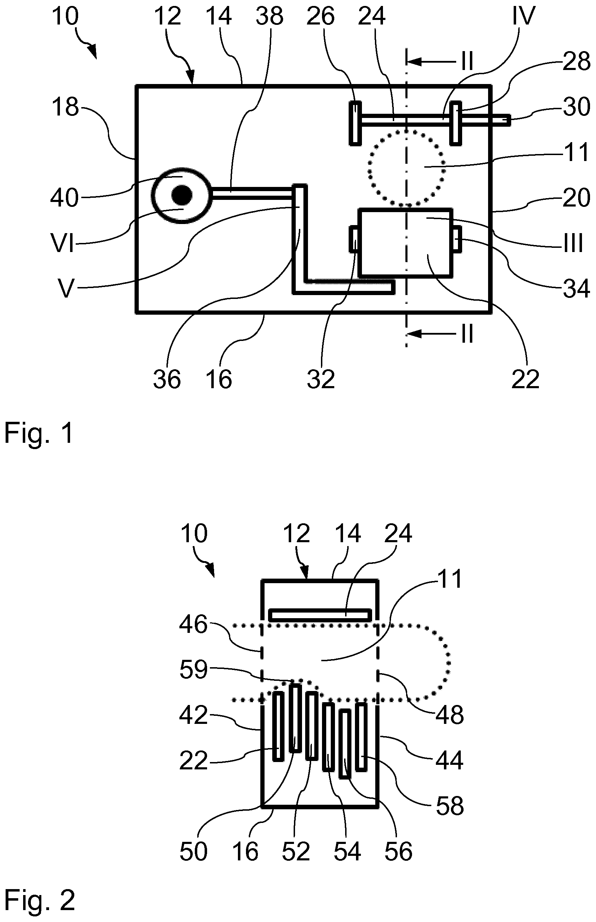

Fig. 1 zeigt eine Schwellkörper-Trainiervorrichtung 10 für einen Schwellkörper in einer Schwellkörper-Position 11. Die Schwellkörper-Position 11 ist hier mit einer gepunkteten Linie, hier in Form einer Kreislinie dargestellt. Die Schwellkörper-Trainiervorrichtung 10 hat ein Gehäuse 12, welches eine Gehäuse-Oberseite 14, eine Gehäuse-Unterseite 16, eine linke Seitenwand 18 und eine rechte Seitenwand 20 aufweist. In Fig. 1 unterhalb der Schwellkörper-Position 11 ist ein Druck-Element 22 angeordnet. Direkt darüber befindet sich ein Gegendruck-Element 24, an dem sich eine linke Führ-Einrichtung 26 und eine rechte Führ-Einrichtung 28 anschließt. In Fig. 1 rechts von der rechten Führ-Einrichtung 28 und aus dem Gehäuse 12 herausragend ist ein Einstell-Knauf 30 angeordnet. Auch an dem Druck-Element 22 befindet sich eine linke Führ-Einrichtung 32 und eine rechte Führ-Einrichtung 34. Links von und unter dem Druck-Element 22 ist ein L-förmiger Kipphebel 36 angeordnet, an den sich auf der linken Seite ein Verbindungs-Element 38 zu einer Zieh-Einrichtung 40 anschließt.

Nachdem die einzelnen Komponenten der Schwellkörper-Trainiervorichtung 10 aufgeführt wurden, erfolgt nun eine Erläuterung zu den jeweiligen Funktionen. Wie bereits erwähnt, ist die Schwellkörper-Position 11 zwischen dem Druck-Element 22 und Gegendruck-Element 24 dafür vorgesehen, den Schwellkörper darin zu positionieren. Dabei deutet die Schwellkörper-Position 11 einen Umfang des Schwellkörpers an. Um den Schwellkörper in seiner Schwellkörper-Position 11 zu fixieren wird das Gegendruck-Element 24 mithilfe des Einstell-Knaufs 30 und entlang der Führ-Einrichtungen 26, 28 derart verschoben, dass der Schwellkörper in seiner Schwellkörper-Position 11 gehalten wird. Das Druck-Element 22 ist dafür vorgesehen, einen wiederkehrenden Druck auf den Schwellkörper auszuüben, indem sich das Druck-Element 22 mithilfe der Führ-Elemente 32, 34 auf und ab bewegt, also in Richtung des Gegendruck-Elements 24 und zurück. Dabei wird das Druck-Element 22 mittels des Kipphebels 36 nach oben gedrückt. Dies geschieht dadurch, dass der Kipphebel 36 mithilfe eines Verbindungs-Elements 38 von einer Zieh-Einrichtung 40 derart gezogen wird, dass sich ein unter dem Druck-Element 22 befindlicher Abschnitt des Kipphebels 36 nach oben bewegt. In Fig. 1 sind nur jeweils ein Druck-Element 22, ein Kipphebel 36 und ein Verbindungs-Element 38 zu sehen. Solche Komponenten 22, 36, 38 sind in diesem Ausführungsbeispiel jeweils sechsfach vorhanden und im Wesentlichen nebeneinander angeordnet.

In Fig. 2 ist die Schwellkörper-Trainiervorrichtung 10 gemäß Schnitt II - II in Fig. 1 abgebildet. Somit sind auch hier das Gehäuse 12, die Gehäuse-Oberseite 14 und die Gehäuse-Unterseite 16, sowie das Druck-Element 22 und das Gegendruck-Element 24 sichtbar. Die Schwellkörper-Position 11 ist wieder mit einer gepunkteten Linie angedeutet. In Fig.2 ist außerdem eine vordere Seitenwand 42 und eine hintere Seitenwand 44 des Gehäuses 12 dargestellt, wobei die vordere Seitenwand 42 eine vordere Wandöffnung 46 und die hintere Seitenwand 44 eine hintere Wandöffnung 48 aufweist. Die Wandöffnungen 46, 48 sind jeweils mittels einer gestrichelten Linie bzw. als gestrichelter Abschnitt der Seitenwände 42, 44 dargestellt. Wie bereits oben erwähnt, hat die Schwellkörper-Trainiervorrichtung 10 in diesem Ausführungsbeispiel sechs Druck-Elemente 22, 50, 52, 54, 56, 58, die im Wesentlichen nebeneinander und unterhalb der Schwellkörper-Position 11 angeordnet sind.

Die Schwellkörper-Position 11 wird von dem Schwellkörper erreicht, indem dieser zuerst in die vordere Wandöffnung 46 und dann durch die hintere Wandöffnung 48 hindurch geschoben wird. Nach einer Fixierung des Schwellkörpers mithilfe des Gegendruck-Elements 24 liegt an dem Schwellkörper ein Druck an, hier verdeutlicht mit einer Innenwölbung 59 auf der den Schwellkörper andeutenden gepunkteten Linie oberhalb der Druck-Elemente 22, 50 und 52. Bei einer Anwendung der Schwellkörper-Trainiervorrichtung 10 bewegt sich jedes einzelne Druck-Element 22, 50, 52, 54, 56, 58 kontinuierlich auf und ab. Dabei erfolgt diese Auf-und-Ab-Bewegung versetzt zueinander, sodass sich oberhalb der Druck-Elemente 22, 50, 52, 54, 56, 58 eine Wellenbewegung in Richtung der hinteren Wandöffnung 48 ergibt.

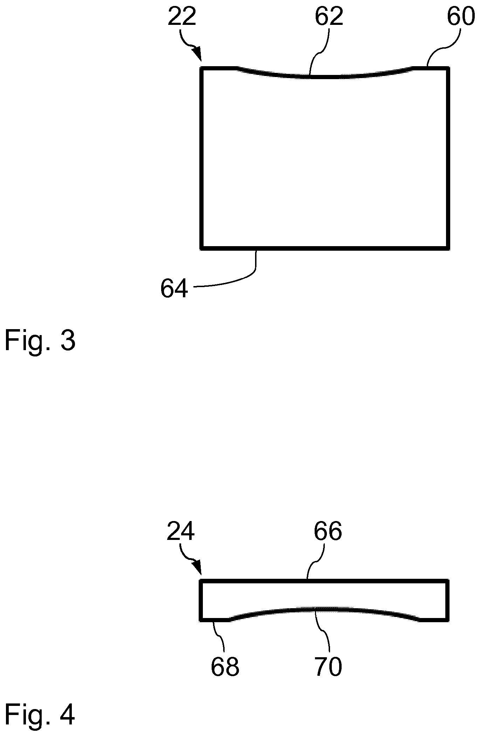

In Fig. 3 ist das Druck-Element 22 gemäß Komponente III in Fig. 1 in einer detaillierteren Form abgebildet. Dabei weist das Druck-Element 22 eine Druck-Element-Oberseite 60 mit einer Innenwölbung 62, sowie eine Druck-Element-Unterseite 64 auf. Mittels der Druck-Element-Oberseite 60 wird der Druck auf den Schwellkörper ausgeübt, stabilisiert mithilfe der Innenwölbung 62. Auf die Druck-Element-Unterseite 64 wirkt bei der Anwendung der Schwellkörper-Trainiervorrichtung 10 eine wiederkehrende Kraft, um das Druck-Element 22 nach oben zu bewegen.

In Fig. 4 ist das Gegendruck-Element 24 gemäß Komponente IV in Fig. 1 in einer detaillierteren Form abgebildet. Das Gegendruck-Element 24 hat eine Gegendruck-Element-Oberseite 66, sowie eine Gegendruck-Element-Unterseite mit einer Innenwölbung 70. Die Gegendruck-Element-Unterseite 68 bewirkt bei der Anwendung der Schwellkörper-Trainiervorrichtung 10 einen Gegendruck auf den Schwellkörper, stabilisiert mittels des Innenwölbung 70.

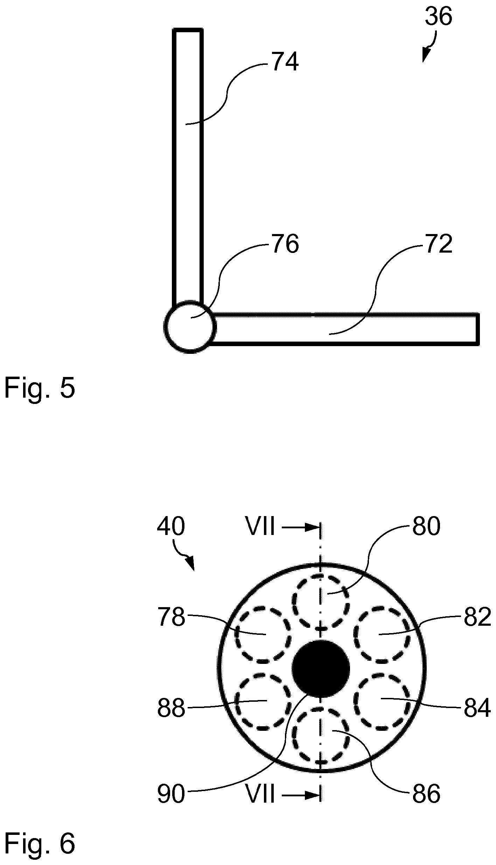

In Fig. 5 ist der Kipphebel 36 gemäß Komponente V in Fig. 1 in einer detaillierteren Form abgebildet. Dabei umfasst der Kipphebel 36 einen Horizontal-Abschnitt 72, einen Vertikal-Abschnitt 74 und eine Drehachse 76. Bei der Anwendung der Schwellkörper-Trainiervorrichtung 10 bewegt sich der Horizontal-Abschnitt 72 nach oben, wodurch eine Kraft auf das Druck-Element 22 ausgeübt wird. Diese Nach-oben-Bewegung geschieht dadurch, dass der Vertikal-Abschnitt 74 in eine Richtung von dem Horizontal-Abschnitt 72 weggezogen wird. Dabei wird der Kipphebel 36 um die Drehachse 76 verdreht. Zur Stabilisierung des Kipphebels 36 ist die Drehachse 76 in dem Gehäuse 12 fest verankert. Wie bereits oben erwähnt, ist ein solcher Kipphebel 36 in diesem Ausführungsbeispiel der Schwellkörper-Trainiervorrichtung sechsmal vorhanden, um die sechs Druck-Elemente 22, 50, 52, 54, 56, 58 zu bewegen.

In Fig. 6 ist die Zieh-Einrichtung 40 gemäß Komponente VI in Fig. 1 in einer detaillierteren Form abgebildet. Die Zieh-Einrichtung 40 ist in diesem Ausführungsbeispiel als eine Rotationswelle gestaltet und beinhaltet sechs Verbindungsbolzen 78, 80, 82, 84, 86, 88, hier als gestrichelte Kreislinien dargestellt, die gleichmäßig um eine Rotationsachse 90 angeordnet sind. Bei der Anwendung der Schwellkörper-Trainiervorrichtung 10 wird die Zieh-Einrichtung 40 bzw. die Rotationswelle 40 gleichmäßig um die Rotationsachse 90 gedreht. Zur Stabilisierung der Rotationswelle 40 ist die Rotationsachse 90 fest in dem Gehäuse 12 verankert.

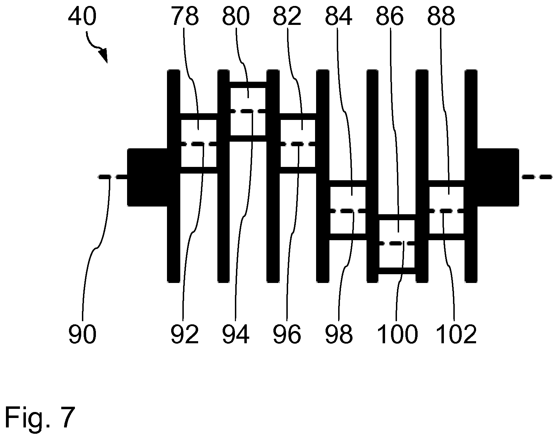

In Fig. 7 ist die Rotationswelle 40 gemäß Schnitt VII - VII in Fig. 6 abgebildet. Dabei wird ersichtlich, dass die Verbindungsbolzen 78, 80, 82, 84, 86, 88 nicht nur um die Rotationsachse 90, sondern auch entlang der Rotationsachse 90 versetzt angeordnet sind. Dabei ist jeder Verbindungsbolzen mit jeweils einer von sechs Verbindungs-Elementen wie das Verbindungs-Element 38 verbunden, die wiederum mit jeweils einem Kipphebel 36 verbunden sind. Die Bolzenachsen 92, 94, 96, 98, 100, 102 der Verbindungsbolzen 78, 80, 82, 84, 86, 88 sind außerhalb der Rotationsachse 90 angeordnet, um bei einer Drehung der Rotationswelle 40 ihren jeweiligen Abstand zu dem jeweiligen Kipphebel 36 zu verändern und diesen somit mittels des jeweiligen Verbindungs-Elements 38 zu ziehen. Die Verbindungsbolzen 78, 80, 82, 84, 86, 88 sind dabei derart angeordnet, dass aus der Drehung der Rotationswelle 40 die Wellenbewegung der Druck-Elemente 22, 50, 52, 54, 56, 58 in Richtung der hinteren Wandöffnung resultiert.

### Bezugszeichenliste

- 10: Schwellkörper-Trainiervorrichtung
- 11: Schwellkörper-Position
- 12: Gehäuse
- 14: Gehäuse-Oberseite
- 16: Gehäuse-Unterseite
- 18: Seitenwand
- 20: Seitenwand
- 22: Druck-Element
- 24: Gegendruck-Element
- 26: Führ-Einrichtung
- 28: Führ-Einrichtung
- 30: Einstell-Knauf
- 32: Führ-Einrichtung
- 34: Führ-Einrichtung
- 36: Kipphebel
- 38: Verbindungs-Element
- 40: Zieh-Einrichtung, Rotationswelle
- 42: Seitenwand
- 44: Seitenwand
- 46: Wandöffnung
- 48: Wandöffnung
- 50: Druck-Element
- 52: Druck-Element
- 54: Druck-Element
- 56: Druck-Element
- 58: Druck-Element
- 59: Innenwölbung
- 60: Druck-Element-Oberseite
- 62: Innenwölbung
- 64: Druck-Element-Unterseite
- 66: Gegendruck-Element-Oberseite
- 68: Gegendruck-Element-Unterseite
- 70: Innenwölbung
- 72: Horizontal-Abschnitt
- 74: Vertikal-Abschnitt
- 76: Drehachse
- 78: Verbindungsbolzen
- 80: Verbindungsbolzen
- 82: Verbindungsbolzen
- 84: Verbindungsbolzen
- 86: Verbindungsbolzen
- 88: Verbindungsbolzen
- 90: Rotationsachse
- 92: Bolzenachse
- 94: Bolzenachse
- 96: Bolzenachse
- 98: Bolzenachse
- 100: Bolzenachse
- 102: Bolzenachse

## Patentansprüche

1. Schwellkörper-Trainiervorrichtung (10) zum Trainieren eines Schwellkörpers eines Penis mit einer Eichel in einer Schwellkörper-Position (11), mit mindestens einem Druck-Element (22) und einem Gegendruck-Element (24), wobei das mindestens eine Druck-Element (22) dafür vorgesehen ist, in Gebrauch in Richtung des Gegendruck-Elements (24) an den zwischen dem mindestens einen Druck-Element (22) und dem Gegendruck-Element (24) angeordneten Schwellkörper einen wiederkehrenden Druck in Form einer Wellenbewegung in Richtung der Eichel anzulegen,
**dadurch gekennzeichnet, dass**
das mindestens eine Druck-Element (22) unterhalb der Schwellkörper-Position (11) angeordnet ist, und das Gegendruck-Element einstellbar ist, um den Schwellkörper in der Schwellkörper-Position zu fixieren.

2. Schwellkörper-Trainiervorrichtung (10) nach Anspruch 1,
**dadurch gekennzeichnet, dass** die Schwellkörper-Trainiervorrichtung (10) von einem Gehäuse (12) mit einer ersten Seitenwand (42) und einer zweiten Seitenwand (44) umgeben ist, wobei die erste Seitenwand (42) eine erste Wandöffnung (46) und die zweite Seitenwand (44) eine zweite Wandöffnung (48) aufweist.

3. Schwellkörper-Trainiervorrichtung (10) nach einem der vorhergehenden Ansprüche,
**dadurch gekennzeichnet, dass** das Gegendruck-Element (24) in seinem Abstand zu dem mindestens einen Druck-Element (22) verstellbar ist, um den Schwellkörper zu fixieren.

4. Schwellkörper-Trainiervorrichtung (10) nach einem der vorhergehenden Ansprüche,
**dadurch gekennzeichnet, dass** das mindestens eine Druck-Element (22) eine Druck-Element-Oberseite (60) und das Gegendruck-Element (24) eine der Druck-Element-Oberseite (60) gegenüberliegende Gegendruck-Element-Unterseite (68) hat, wobei die Druck-Element-Oberseite (60) und die Gegendruck-Element-Unterseite (68) jeweils eine Innenwölbung (62, 70) aufweisen.

5. Schwellkörper-Trainiervorrichtung (10) nach einem der vorhergehenden Ansprüche,
**dadurch gekennzeichnet, dass** die Schwellkörper-Trainiervorrichtung (10) ferner mindestens einen Kipphebel (36) aufweist, der zum Anlegen eines Drucks an das mindestens eine Druck-Element (22) vorgesehen ist.

6. Schwellkörper-Trainiervorrichtung (10) nach Anspruch 5,
**dadurch gekennzeichnet, dass** der mindestens eine Kipphebel (36) L-förmig mit einem Horizontal-Abschnitt (72) und einem Vertikal-Abschnitt (74) gestaltet ist, wobei der Horizontal-Abschnitt (72) vorzugsweise zum Drücken des mindestens einen Druck-Elements (22) in Richtung des Gegendruck-Elements (24) vorgesehen ist.

7. Schwellkörper-Trainiervorrichtung (10) nach Anspruch 6,
**dadurch gekennzeichnet, dass** der mindestens eine Kipphebel (36) mit einer Drehachse (76) verbunden ist, wobei die Drehachse (76) rechtwinklig zum Horizontal-Abschnitt (72) und zum Vertikal-Abschnitt (74) des mindestens einen Kipphebels (36) angeordnet ist.

8. Schwellkörper-Trainiervorrichtung (10) nach einem der Ansprüche 5 bis 7, **dadurch gekennzeichnet, dass** die Schwellkörper-Trainiervorrichtung (10) ferner eine Zieh-Einrichtung (40) aufweist, wobei die Zieh-Einrichtung (40) mittels mindestens eines Verbindungs-Elements (38) mit dem mindestens einen Kipphebel (36) verbunden ist.

9. Schwellkörper-Trainiervorrichtung (10) nach Anspruch 8,
**dadurch gekennzeichnet, dass** die Zieh-Einrichtung (40) als eine Rotationswelle mit mindestens einem Verbindungsbolzen (78, 80, 82, 84, 86, 88) gestaltet ist, wobei der mindestens eine Verbindungsbolzen (78, 80, 82, 84, 86, 88) mit dem mindestens einen Verbindungs-Element (38) verbunden ist und eine Bolzenachse (92, 94, 96, 98, 100, 102) des mindestens einen Verbindungsbolzen (78, 80, 82, 84, 86, 88) außerhalb einer Rotationsachse (90) der Rotationswelle angeordnet ist.

10. Schwellkörper-Trainiereinrichtung (10) nach Anspruch 9,
**dadurch gekennzeichnet, dass** die Rotationswelle mehrere Verbindungsbolzen (78, 80, 82, 84, 86, 88) aufweist, die um die Rotationsachse (90) der Rotationswelle derart angeordnet sind, dass sie eine Spiralform bilden.

## Claims

1. An erectile tissue training device (10) for training an erectile tissue of a penis with a glans in an erectile tissue position (11), comprising at least one pressure element (22) and a counter-pressure element (24), wherein the at least one pressure element (22) is provided to apply, in use, in the direction of the counter-pressure element (24) to the erectile tissue arranged between the at least one pressure element (22) and the counter-pressure element (24), a recurring pressure in the form of a wave motion in the direction of the glans,
**characterized in that**
the at least one pressure element (22) is arranged below the erectile tissue position (11), and the counter-pressure element is adjustable to fix the erectile tissue in the erectile tissue position.

2. The erectile tissue training device (10) according to claim 1,
**characterized in that** the erectile tissue training device (10) is surrounded by a housing (12) with a first side wall (42) and a second side wall (44), wherein the first side wall (42) has a first wall opening (46) and the second side wall (44) has a second wall opening (48).

3. The erectile tissue training device (10) according to any one of the preceding claims,
**characterized in that** the distance of the counter-pressure element (24) to the at least one pressure element (22) is adjustable to fix the erectile tissue.

4. The erectile tissue training device (10) according to any one of the preceding claims,
**characterized in that** the at least one pressure element (22) has a pressure element top side (60) and the counter-pressure element (24) has a counter-pressure element bottom side (68) opposite the pressure element top side (60), wherein the pressure element top side (60) and the counter-pressure element bottom side (68) each have an internal curvature (62, 70).

5. The erectile tissue training device (10) according to any one of the preceding claims,
**characterized in that** the erectile tissue training device (10) further comprises at least one rocker arm (36) which is provided for applying a pressure to the at least one pressure element (22).

6. The erectile tissue training device (10) according to claim 5,
**characterized in that** the at least one rocker arm (36) is L-shaped with a horizontal section (72) and a vertical section (74), wherein the horizontal section (72) is preferably provided for pressing the at least one pressure element (22) in the direction of the counter-pressure element (24).

7. The erectile tissue training device (10) according to claim 6,
**characterized in that** the at least one rocker arm (36) is connected to an axis of rotation (76), wherein the axis of rotation (76) is arranged at a right angle to the horizontal section (72) and to the vertical section (74) of the at least one rocker arm (36).

8. The erectile tissue training device (10) according to any one of claims 5 to 7,
**characterized in that** the erectile tissue training device (10) further comprises a pulling device (40), wherein the pulling device (40) is connected to the at least one rocker arm (36) by means of at least one connecting element (38).

9. The erectile tissue training device (10) according to claim 8,
**characterized in that** the pulling device (40) is designed as a rotating shaft with at least one connecting pin (78, 80, 82, 84, 86, 88), wherein the at least one connecting pin (78, 80, 82, 84, 86, 88) is connected to the at least one connecting element (38) and a pin axis (92, 94, 96, 98, 100, 102) of the at least one connecting pin (78, 80, 82, 84, 86, 88) is arranged outside a rotation axis (90) of the rotating shaft.

10. The erectile tissue training device (10) according to claim 9,
**characterized in that** the rotating shaft has a plurality of connecting pins (78, 80, 82, 84, 86, 88) which are arranged around the rotation axis (90) of the rotating shaft in such a way that they form a spiral shape.

## Revendications

1. Dispositif d'entraînement pour corps caverneux (10) destiné à entraîner un corps caverneux d'un pénis avec un gland dans une position de corps caverneux (11), comprenant au moins un élément de pression (22) et un élément de contre-pression (24), dans lequel ledit au moins un élément de pression (22) est prévu pour appliquer, en utilisation, une pression répétitive en direction de l'élément de contre-pression (24), sous la forme d'un mouvement ondulatoire en direction du gland sur le corps caverneux situé entre ledit au moins un élément de pression (22) et ledit élément de contre-pression (24),
**caractérisé en ce que** ledit au moins un élément de pression (22) est disposé en dessous de la position du corps caverneux (11), et l'élément de contre-pression est réglable afin de fixer le corps caverneux dans la position du corps caverneux.

2. Dispositif d'entraînement pour corps caverneux (10) selon la revendication 1, **caractérisé en ce que** le dispositif d'entraînement pour corps caverneux (10) est entouré d'un boîtier (12) comportant une première paroi latérale (42) et une deuxième paroi latérale (44), la première paroi latérale (42) présentant une première ouverture de paroi (46) et la deuxième paroi latérale (44) présentant une deuxième ouverture de paroi (48).

3. Dispositif d'entraînement pour corps caverneux (10) selon l'une des revendications précédentes,
**caractérisé en ce que** l'élément de contre-pression (24) est réglable dans sa distance par rapport audit au moins un élément de pression (22) afin de fixer le corps caverneux.

4. Dispositif d'entraînement pour corps caverneux (10) selon l'une des revendications précédentes,
**caractérisé en ce que** ledit au moins un élément de pression (22) présente une face supérieure d'élément de pression (60) et l'élément de contre-pression (24) présente une face inférieure d'élément de contre-pression (68) opposée à la face supérieure d'élément de pression (60), la face supérieure d'élément de pression (60) et la face inférieure d'élément de contre-pression (68) présentant chacune un galbe concave (62, 70).

5. Dispositif d'entraînement pour corps caverneux (10) selon l'une des revendications précédentes,
**caractérisé en ce que** le dispositif d'entraînement pour corps caverneux (10) comprend en outre au moins un levier basculant (36) prévu pour appliquer une pression sur ledit au moins un élément de pression (22).

6. Dispositif d'entraînement pour corps caverneux (10) selon la revendication 5, **caractérisé en ce que** ledit au moins un levier basculant (36) est conçu en forme de L avec une partie horizontale (72) et une partie verticale (74), la partie horizontale (72) étant prévue de préférence pour pousser ledit au moins un élément de pression (22) en direction de l'élément de contre-pression (24).

7. Dispositif d'entraînement pour corps caverneux (10) selon la revendication 6, **caractérisé en ce que** ledit au moins un levier basculant (36) est relié à un axe de rotation (76), l'axe de rotation (76) étant disposé perpendiculairement à la partie horizontale (72) et à la partie verticale (74) dudit au moins un levier basculant (36).

8. Dispositif d'entraînement pour corps caverneux (10) selon l'une des revendications 5 à 7,
**caractérisé en ce que** le dispositif d'entraînement pour corps caverneux (10) comprend en outre un équipement de traction (40), l'équipement de traction (40) étant relié audit au moins un levier basculant (36) au moyen dudit au moins un élément de liaison (38).

9. Dispositif d'entraînement pour corps caverneux (10) selon la revendication 8, **caractérisé en ce que** l'équipement de traction (40) est conçu comme un arbre rotatif avec au moins un boulon de liaison (78, 80, 82, 84, 86, 88), ledit au moins un boulon de liaison (78, 80, 82, 84, 86, 88) étant relié audit au moins un élément de liaison (38) et **en ce qu'**un axe de boulon (92, 94, 96, 98, 100, 102) dudit au moins un boulon de liaison (78, 80, 82, 84, 86, 88) est disposé à l'extérieur d'un axe de rotation (90) de l'arbre rotatif.

10. Dispositif d'entraînement pour corps caverneux (10) selon la revendication 9, **caractérisé en ce que** l'arbre rotatif comporte plusieurs boulons de liaison (78, 80, 82, 84, 86, 88) qui sont disposés autour de l'axe de rotation (90) de l'arbre rotatif de manière à constituer une forme hélicoïdale.
